(19) Europäisches Patentamt
European Patent Office
Office européen des brevets

(11) Publication number: **0 154 474**
**B1**

(12) # EUROPEAN PATENT SPECIFICATION

(45) Date of publication of patent specification: **09.11.88**

(51) Int. Cl.[4]: **C 07 F 9/65,** C 07 D 295/12, C 02 F 5/14, C 04 B 24/00

(21) Application number: **85301237.5**

(22) Date of filing: **25.02.85**

(54) **Bis(aminoalkyl)piperazine derivatives and their use as metal ion control agents and cement set retarding agents.**

(30) Priority: **24.02.84 US 583526**
**24.02.84 US 583527**

(43) Date of publication of application:
**11.09.85 Bulletin 85/37**

(45) Publication of the grant of the patent:
**09.11.88 Bulletin 88/45**

(84) Designated Contracting States:
**DE FR GB NL**

(56) References cited:
**SU-A- 905 434**
**US-A-3 674 804**

(73) Proprietor: **THE DOW CHEMICAL COMPANY**
**2030 Dow Center Abbott Road P.O. Box 1967**
**Midland, MI 48640 (US)**

(72) Inventor: **Crump, Druce K.**
**142 Oyster Creek Drive No. 48**
**Lake Jackson Texas 77566 (US)**
Inventor: **Simon, Jaime**
**Rt. 1 Box 120-G**
**Angleton Texas 77515 (US)**
Inventor: **Wilson, David A.**
**229 San Saba**
**Richwood Texas 77531 (US)**

(74) Representative: **Burford, Anthony Frederick et al**
**W.H. Beck, Greener & Co. 7 Stone Buildings**
**Lincoln's Inn**
**London WC2A 3SZ (GB)**

**EP 0 154 474 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The use of methylenephosphonic acid substituted alkylene polyamines for metal ion control at less than stoichiometric amounts was suggested in a patent to Bersworth (US—A—2,609,390) in 1952. Later a water-dispersible polymeric amine chelating agent which included alkylene phosphonate derivatives was indicated as having "threshold" effects in scale inhibition applications (US—A—3,331,773), this term being used to describe the use of the agent in less than stoichiometric amounts. The diamine and polyamine methylenephosphonate derivatives are taught and claimed in US—A—3,336,221 and US—A—3,434,969, respectively. Some of the products disclosed in these two patents are available commercially and are recommended as scale inhibitors when applied in threshold amounts.

Some other patents which disclose heterocyclic nitrogen-containing compounds which are useful as chelating agents and may be employed in threshold amounts are US—A—3,674,804; US—A—3,720,498; US—A—3,743,603; US—A—3,859,211; and US—A—3,954,761. Some of the compounds included therein are heterocyclic compounds having the formulas:

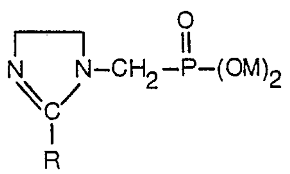

wherein R is hydrogen or alkyl and M is hydrogen, alkali metal, ammonium or a di- or triethanolamine radical;

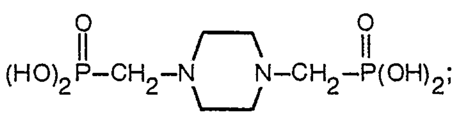

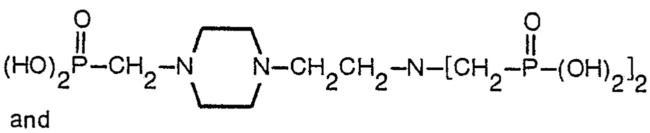

and

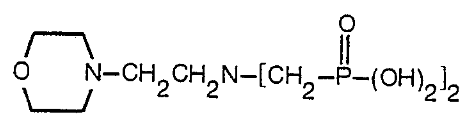

Methylenephosphonates of polyalkylene polyamines, disclosed in US—A—4,051,110, are made by reacting di- or polyamines with a chain-extending agent such as a dihalide or an epoxyhalide, e.g., ethylene dichloride or epichlorohydrin and thereafter, with phosphorus acid and formaldehyde. Thus, for example, triethylenetetramine is reacted with epichlorohydrin in an approximately one to one mole ratio; thereafter the product is reacted with phosphorus acid, and formaldehyde in the presence of hydrochloric acid. The resulting methylenephosphonated polyamine is useful in small amounts as a scale inhibitor, being employed at concentrations of 20—50 ppm.

Certain phosphonic acid derivatives of the aliphatic acids can be prepared by reacting phosphorous acid with acid anhydrides or acid chlorides, e.g., the anhydrides or chlorides of acetic, propionic and valeric acids. The compounds prepared have the formula

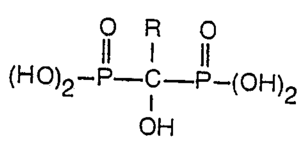

wherein R is a lower alkyl radical having 1 to 5 carbon atoms. The method of making and use of these products is described in US—A—3,214,454. It discloses and claims the use of threshold amounts to prevent calcium precipitation in aqueous solutions.

It has now been found that certain phosphonate derivatives of bis(aminoalkyl)piperazines are good threshold agents to prevent metal ion precipitation in aqueous solutions. They also function as sequestering and or chelating agents as do those which do not contain the phosphonate group.

While the trimethylenephosphonate of aminoethylpiperazine itself has been shown not to have very good threshold activity, the related derivatives of bis(aminoalkyl)piperazine are quite effective.

The compounds from which the methylenephosphonates are derived have the formula I:

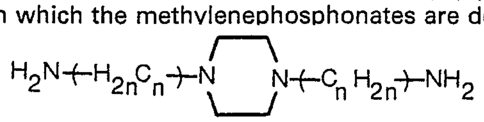

(I)

wherein n is 2 or 3.

These compounds can be made from piperazine and acrylonitrile or glycolonitrile, followed by reduction to the amine. The bis(aminopropyl) compound is commercially available.

The bis-amine is then reacted with phosphorus acid and formaldehyde (or a source of formaldehyde) as shown in Example 1 below. This phosphonomethylation is known to the art and is shown in a number of patents including US—A—3,336,221 and US—A—4,051,110.

The compounds which are the subject of the present invention have the formula II:

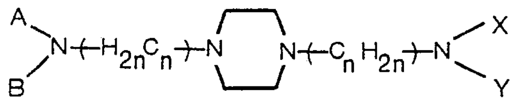

(II)

wherein n is 2 or 3 and wherein substituents A, B, X and Y each are independently selected from radicals consisting of hydrogen, hydroxyalkyl (wherein the alkyl group contains 2—6 carbon atoms), methylenephosphonic, methylene-, ethylene- and propylene-sulfonic, hydroxymethyl-, hydroxyethyl- and hydroxypropyl-sulfonic acid radicals, carboxylic acid radicals (having 2—4 carbon atoms) and the alkali or alkaline earth metal, ammonia and amine salts of any of the phosphonic, sulfonic or carboxylic acid derivatives. At least one of A, B, X and Y must be other than a hydrogen.

All the above substituents for the hydrogens of the amine groups of the above bis amine compounds form useful chelating agents, but only the methylenephosphonic acid-substituted compounds and their alkali or alkaline earth metal, ammonium or amine salt derivatives are effective as threshold agents.

The present invention also pertains to aqueous hydraulic cement slurry compositions containing methylene phosphonic acid derivatives of bis(aminoalkyl)piperazines of the above formula as set retarding agents.

Hydrophobic-substituted phosphonic or phosphinic acids and their alkali metal salts have been used in cements, primarily soil cement mixtures, to improve the freeze-thaw properties and salt-resistance. Six- to eighteen-carbon alkyl phosphonic acids or their alkali metal salts are so described in US—A—3,794,506. A plugging mixture for high temperature oil and gas wells comprising Portland cement and 1-hydroxy ethylidenephosphonic acid trisodium or tripotassium salts as set time extenders is described in Derwent abstract 71376B/39 (1979) of SU—A—640,019. The use of these phosphonate salts at temperatures of 75°C to 150°C in amounts of 0.1—0.3 percent by weight is described in the abstract.

Other organic phosphorus acid derivatives are taught to be useful additives in cement compositions as turbulence-inducing and flow-property improver additives (US—A—3,964,921 and US—A—4,040,854, respectively). Another turbulence-inducer is a pyrolysis product of urea and a bis(alkylenepyrophosphate) (US—A—3,409,080).

Alkylene diphosphonic acids and their water-soluble salts are described as set time extenders and water reducing agents for gypsum plasters (US—A—4,225,361). Lignins which have been phosphonoalkylated through an ether linkage or corresponding sulfonates, sulfides, hydroxyl or amine derivatives are taught to be useful primarily as dispersants or surfactants (US—A—3,865,803) and are also said to be useful as "cement additives" without indicating specific uses.

Ultra-rapid hardening Portland cement compositions are described which contain various acid salt additives (US—A—4,066,469). It states that use of acid phosphates as the acid salt additives is excluded since the phosphates have a characteristically powerful retarding property peculiar to them.

Most of the cement used in oil wells is called Portland cement. Portland cement is manufactured by calcining raw materials consisting of limestone, clay, shale, and slag together at 2,600°F (1,426.8°C) to 2,800°F (1,537.7°C) in a rotary kiln.

The resulting material is cooled and interground with small percentages of gypsum to form Portland cement. In addition to the above raw materials, ether components such as sand, bauxite, iron oxide, etc., may be added to adjust the chemical composition depending upon the type of Portland cement desired.

The principal components of the finished Portland cement are lime, silica, alumina, and iron. These components form the following complex compounds: tricalcium aluminate, ($3CaO \cdot Al_2O_3$), tetracalcium aluminoferrite, ($4CaO \cdot Al_2O_3 \cdot Fe_2O_3$), tricalcium silicate, ($3CaO \cdot SiO_2$), and dicalcium silicate, ($2CaO \cdot SiO_2$).

When water is added to cement, setting and hardening reactions begin immediately. The chemical compounds in the cement undergo the processes of hydration and recrystallization which result in a set product. The maximum amount of water that can be used with an oil-well cement is the amount which can be added before solids separation occurs. The minimum amount of water is the amount required to make the slurry pumpable. Therefore, the normal water ratio is governed by the maximum and minimum limits for a particular class of cement.

Thickening time is the time that the cement remains pumpable in the well. This is the most critical property of an oil-well cement. The thickening time has to be long enough to be pumped into place and short enough to permit operations to resume quickly. Generally, three hours provides the necessary placement time plus a safety factor.

Other factors, such as fluid loss, viscosity and density must be taken into consideration and additives are known to the art-skilled which affect each of these factors as well as that of set, or thickening, time as mentioned above. Another parameter which has an effect on set time is temperature. Cement sets more

rapidly as the temperature increases. This must be taken into consideration particularly when pumping cement into deeper wells since temperature increases as the depth of the well becomes greater. Temperature also affects the strength of the cement, the strength becoming less as the temperature increases.

Because of this temperature effect, it is important to retard the setting of the cement employed in the deeper wells.

It has now been discovered that certain new phosphonomethylated bis(aminoalkyl)piperazines of the present invention are useful in aqueous cement slurries as set retarding additives. Some of these compounds are chelating agents, while others are useful as threshold agents in retarding the precipitation of metal ions from aqueous solution. However, all such compounds which are useful as cement set-retarders must contain at least one methylenephosphonate group.

Those compounds of the present invention which are useful as cement retarders in aqueous cement slurries are methylene phosphonic acid derivatives having the following formula:

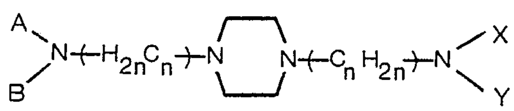

wherein n is 2—3 and wherein substituents A, B, X and Y each are independently selected from radicals including hydrogen; hydroxyalkyl (wherein the alkyl group contains 2—6 carbon atoms); methylenephosphonic; methylene-, ethylene- and propylenesulfonic; hydroxymethyl-, hydroxyethyl- and hydroxypropylsulfonic acid radicals; carboxylic acid radicals (having 2—4 carbon atoms) and the alkali or alkaline earth metal; ammonium and amine salts of any of the phosphonic, sulfonic or carboxylic acid derivatives. At least one of A, B, X and Y must be a methylenephosphonic acid radical or a salt thereof.

The compounds of the present invention are prepared from the corresponding bis(aminoalkyl)piperazine of formula I using methods known *per se* for substituting amino groups with hydroxyalkyl, methylenephosphonic, alkylenesulfonic, hydroxyalkylsulphonic or carboxylic and radicals.

For example, one or more hydroxyalkyl radicals can be introduced into a compound of formula I, or of formula II in which at least one of A, B, X and Y is hydrogen, by reaction of said compound with an epoxide as described in US—A—3,398,198.

As previously described, one or more methylenephosphonic radicals can be introduced into a compound of formula I, or of formula II in which at least one of A, B, X and Y is hydrogen, by reaction of said compound with phosphorus acid and formaldehyde (or a source of formaldehyde) as described in US—A—3,336,221 and US—A—4,051,110.

One or more methylenesulfonic radicals can be introduced into a compound of formula I, or of formula II in which at least one of A, B, X and Y is hydrogen, by reaction with sodium bisulfite and formaldehyde as described in "Preparation and Properties of Aminomethylenesulfonic Acids" (J. Am. Chem. Soc. *77*, 5512-15, 1955). Other alkylsulfonic radicals can be introduced by reaction of said bis amine reactant with the appropriate chloroalkyl-sulfonic acid as described in US—A—4,085,134.

One or more hydroxymethylsulfonic or hydroxyethylsulfonic radicals can be introduced into a compound of formula I, or formula II in which at least one of A, B, X and Y is hydrogen, by the method described in US—A—3,726,912.

One or more hydroxypropylsulfonic radicals can be introduced into a compound of formula I, or formula II in which at least one of A, B, X and Y is hydrogen, by reaction in aqueous solution with 3-chloro-2-hydroxy-1-propanesulfonic acid in the presence of caustic, e.g. sodium hydroxide, and, if the acid form is required, subsequent acidification with a strong acid, e.g. hydrochloric acid. The reaction is described in US—A—3,091,522.

One or more carboxylic radicals can be introduced into a compound of formula I, or of formula II in which at least one of A, B, X and Y is hydrogen, by reaction with an appropriate nitrile, e.g. glycolonitrile or acrylonitrile, in the presence of caustic, e.g. sodium hydroxide and, if the acid form is required, subsequent acidification. The reaction is described in US—A—3,726,912.

The salts of the acid derivatives of formula II can be prepared from the free acids in conventional manner.

The following Examples illustrate further the present invention.

Example 1

Deionized water (10 g) and 20.0 g (0.10 mole) of bis(aminopropyl)piperazine were weighed into a 250-ml round-bottom reaction flask equipped with a water-cooled reflux condenser, mechanical stirrer, thermometer with a temperature controller, and an addition funnel. Approximately 40 g of concentrated HCl solution and 38.5 g (0.47 mole) of phosphorous acid were added to the aqueous amine solution and the reaction mixture heated to reflux and maintained for one hour. Aqueous 37 percent formaldehyde solution (34.0 g—0.42 mole) was added to the addition funnel and added over a one-hour period. The reaction mixture was heated at reflux for an additional 4 hours and then cooled. The product was the completely phosphonomethylated bis(aminopropyl)piperazine.

In like manner other experiments were conducted except that different amounts of reactants were used and some of the products were neutralised with caustic. Conditions are listed in Table I below.

TABLE 1

| Example* | Bis amine | (g) Amount | (g) HCl | (mole) $H_3PO_3$ | (mole) HCHO | NaOH |
|---|---|---|---|---|---|---|
| 2 | aminopropyl | 20 | 40 | 0.35 (28.7 g) | 0.32 (9.6 g) | — |
| 3 | aminoethyl | 17.2 | 40 | 0.47 (38.5 g) | 0.42 (12.6 g) | neut. |
| 4 | aminoethyl | 17.2 | 40 | 0.35 (28.7 g) | 0.32 (9.6 g) | neut. |

*The products are identified as follows: Example 2 contained three mole equivalents of methylenephosphonic acid functionality and one mole equivalent of hydrogen functionality. Example 3 contained four mole equivalents of methylenephosphonic acid functionality in the sodium form. Example 4 contained the same functionalities and ratios as in Example 2.

Example 5

The procedure of Example 4 was repeated and the reaction product carboxymethylated using 0.12 mole (6.8 g) of aqueous glycolonitrile ($HOCH_2C{\equiv}N$) in the presence of excess caustic to produce the sodium salt. The product contained an average of one mole equivalent of carboxymethyl functionality and three mole equivalents of methylenephosphonic acid functionality, all in the sodium form.

Example 6

Deionized water (15.2 g) and 16.2 g (0.094 mole) of bis(aminoethyl)piperazine were weighed into a 250-ml round-bottom reaction flask equipped with a water-cooled reflux condenser, mechanical stirrer, thermometer with a temperature controller, and an addition funnel. Caustic solution (2.2 g of 50 percent) and 18.7 g (0.095 mole) of 3-chloro-2-hydroxy-1-propanesulfonic acid, sodium salt were added with stirring and the reaction mixture heated at 90°C for 2 hours. Approximately 43 g of concentrated HCl solution and 33.1 g (0.28 mole) of 70 percent aqueous phosphorous acid solution were added and the reaction mixture heated to reflux and maintained for one hour. Aqueous 37 percent formaldehyde solution (22.9 g — 0.28 mole) was added to the addition funnel and added over about a one-hour period. The reaction mixture was heated at reflux for an additional $2\frac{1}{2}$ hours and then cooled. The final product solution was neutralized with potassium hydroxide solution. It contained an average of one mole equivalent of hydroxypropylsulfonate functionality and 3 mole equivalents of methylenephosphonate functionality.

Example 7

The procedure of Example 6 was followed using 18.8 g (0.094 mole) of bis(aminopropyl)piperazine. The final product solution was not neutralized. It contained the same functional groups and ratios as Example 6 above, but derived from the bis(aminopropyl)piperazine, in the acid rather than salt form.

Example 8

Propylene oxide (7 g — 0.12 mole) was reacted with 16.2 g (0.094 mole) of bis(aminoethyl)piperazine and the reaction product then phosphonomethylated according to the procedure of Example 1 using 0.28 mole of phosphorous acid and formaldehyde solution. The final product solution was neutralized with KOH solution. It contained an average of one mole equivalent of hydroxypropyl functionality and 3 mole equivalents of methylenephosphonate functionality.

Example 9

Bis(aminoethyl)piperazine was reacted with glycolonitrile in the presence of excess caustic to produce the tetrasodium salt of bis(aminoethyl)piperazine-tetraacetic acid.

Example A (Comparative)

Aminoethyl piperazine (AEP) was phosphonomethylated to obtain aminoethylpiperazine trimethylenephosphonic acid, the completely phosphonated AEP.

To show the utility of the above compounds as scale inhibitors, the following test was run.

Calcium Scale Inhibitor Test

The compounds were evaluated as scale inhibitors for calcium carbonate scale according to National Association of Corrosion Engineers (NACE) test method TM-03-74. The results are shown in Table II. Note

the dramatic improvement of the phosphonic acids prepared from the bis(aminoethyl)piperazine and bis(aminopropyl)piperazine compounds of this invention when compared to aminoethylpiperazine-trimethylenephosphonic acid taught in the U.S. patent literature as a good scale inhibition compound.

TABLE II
Scale Inhibition Data

| Compound | Concentration* (ppm) | Percent Inhibition at 24 Hr | 48 Hr | 72 Hr |
|---|---|---|---|---|
| Example A (comparative) | 10 | 77 | 65 | 60 |
| Example 1 | 10 | 99 | 93 | 90 |
| Example 2 | 10 | 99 | 93 | 91 |
| Example 3 | 10 | 99 | 91 | 89 |
| Example 4 | 10 | 97 | 87 | 79 |
| Example 5 | 10 | 96 | 89 | 83 |
| Example 6 | 10 | 85 | 73 | 75 |
| Example 7 | 10 | 92 | 77 | 79 |
| Example 8 | 10 | 79 | 77 | 73 |
| Example 9** | 10 | 55 | 51 | 50 |
| Blank (none) | — | 52 | 51 | 50 |

*ppm based on active acid content
**this is not a good threshold compound, but see below for utility as chelating or sequestering agent

The compounds of this invention can also function as sequestering/chelating agents. For example, compounds were titrated with standard copper solution in the presence of chrome azurol-S indicator. The compound of Example 9 complexed about one mole of copper per mole of ligand. Thus, the compound of Example 9, although ineffective as a threshold compound, can be utilized as a chelating agent. The compound of Example 3 was titrated and found to complex approximately 3 moles of copper per mole of ligand. The compound can function as either a threshold compound or a chelating agent.

The compounds of the invention which contain one or more methylenephosphonic acid radicals as substituents on the primary amine nitrogens can be used as threshold agents to prevent the precipitation of metal ions, e.g., $Ca^{++}$, $Mg^{++}$, $Ba^{++}$, and the like. They are employed at substoichiometric amounts based on the metal ions present, the precipitation of which is to be prevented.

These and the remaining compounds which do not contain the methylene phosphonate radical may be employed as chelating agents when used at or near stoichiometric quantities.

The compounds prepared in Examples 1—9 were also tested for retarding the settings of cement according to the following procedure:

Retarder Screening Procedure

1. The following ingredients were weighed:
   cement — 100 g
   water — 38 g
   additive — 0.2 g active
2. Water and liquid additive were mixed.
3. Cement added to the liquid, bottle closed tightly and mixed by shaking.
4. Bottle placed in a preheated 180°F (82°C) bath.
5. Setting of cement checked after 6 and 24 hours.

A blank was always run without the additive for comparison. The compounds, identified by reference to the formula in the Summary above, and the results of the above test are shown in Table III.

The compounds of the invention are particularly advantageous when used in cement slurries placed in oil wells wherein the temperature is 180°F (82°C) or above. Many of the known cement retarding additives are operable only at lower temperatures and frequently decompose at the higher temperatures at which the present additives work to retard the setting of cement.

TABLE III

| | | Additive Compound | | | | Time | |
|---|---|---|---|---|---|---|---|
| Ex. | n | A | B | X | Y | 6 Hr | 24 Hr |
| 1 | 3 | $—CH_2PO_3H_2$ | $—CH_2PO_3H_2$ | $—CH_2PO_3H_2$ | $—CH_2PO_3H_2$ | retarding, not set | retarding, not set |
| 2 | 3 | " | " | " | H | retarding, not set | retarding, not set |
| 3* | 2 | " | " | " | $—CH_2PO_3H_2$ | retarding, not set | retarding, not set |
| 4* | 2 | " | " | " | H | retarding, not set | retarding, not set |
| 5* | 2 | " | " | " | $—CH_2COOH$ | retarding, not set | retarding, not set |
| 6* | 2 | " | " | " | $—CH_2CH(OH)—CH_2SO_3H$ | retarding, not set | retarding, not set |
| 7 | 3 | " | " | " | " | retarding, not set | retarding, not set |
| 8* | 2 | " | " | " | $—CH_2CH(OH)-CH_3$ | retarding, not set | retarding, not set |
| 9* | 2 | $—CH_2COOH$ | $—CH_2COOH$ | $—CH_2COOH$ | $—CH_2COOH$ | set | set |
| Blank | | | —none— | | | set | set |

*neutralized with aqueous caustic solution.

**Claims**

1. A compound having the formula

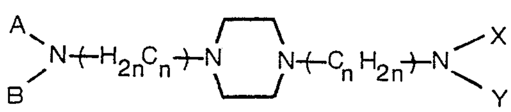

wherein n is 2 or 3 and wherein substituents A, B, X and Y each are independently selected from hydrogen, hydroxyalkyl (wherein the alkyl group contains 2—6 carbon atoms), methylenephosphonic, methylene-, ethylene- and propylene-sulfonic, hydroxymethyl-, hydroxyethyl- and hydroxypropyl sulfonic acid radicals, carboxylic acid radicals (having 2—4 carbon atoms) and the alkali or alkaline earth metal, ammonium and amine salts of any of the phosphonic, sulfonic or carboxylic acid derivatives, and wherein at least one of A, B, X and Y is other than a hydrogen.

2. A compound as claimed in Claim 1, wherein at least one of A, B, X and Y is a methylenephosphonic acid radical or a salt thereof.

3. A compound as claimed in Claim 2, wherein each of A, B, X and Y is a methylenephosphonic acid radical or a salt thereof.

4. A compound as claimed in Claim 2, wherein each of three of A, B, X and Y is a methylenephosphonic acid radical or a salt thereof and the remaining one is hydrogen.

5. A compound as claimed in Claim 2, wherein each of three of A, B, X and Y is a methylenephosphonic acid radical or a salt thereof and the remaining one is a 2-hydroxypropyl-3-sulfonic acid radical or a salt thereof.

6. A compound as claimed in Claim 2, wherein each of three of A, B, X and Y each are a methylenephosphonic acid radical or a salt thereof and the remaining one is a carboxymethyl radical or a salt thereof.

7. A compound as claimed in Claim 2, wherein each of three of A, B, X and Y is a methylenephosphonic acid radical or a salt thereof and the remaining one is a 2-hydroxypropyl radical.

8. A compound as claimed in Claim 1, wherein each of A, B, X and Y is a carboxymethyl radical or a salt thereof.

9. A compound as claimed in any of Claims 2 to 8, wherein all the acid groups are in salt form.

10. A compound as claimed in any one of Claims 2 to 9, in the form of an alkali metal or alkaline earth metal salt.

11. A compound as claimed in Claim 10, wherein the salt is a sodium salt.

12. A process for inhibiting the precipitation of metal ions from their aqueous solutions by adding to said solutions in less than stoichiometric amounts, based on the metal ions present, a phosphonic acid derivative, characterised in that said derivative is a compound as claimed in any one of Claims 2 to 7 and 9 to 11.

13. A process for chelating metal ions in an aqueous solution thereof by adding thereto at least a stoichiometric amount based on the metal ions present of an amine derivative, characterised in that said derivative is a compound as claimed in any one of Claims 1 to 11.

14. A process for retarding the setting of an aqueous cement slurry which comprises adding to said slurry an organic phosphonate, characterised in that said phosphonate is a compound as claimed in any one of Claims 2 to 7 and 9 to 11.

15. A process as claimed in Claim 14, wherein the temperature of the cement slurry is at least 80°C (180°F).

16. A process as claimed in Claim 14 or Claim 15, wherein the cement slurry is injected into an oil well.

**Patentansprüche**

1. Verbindung der Formel

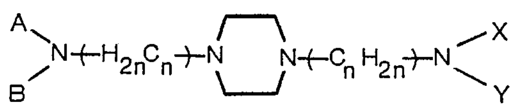

worin n 2 oder 3 und worin die Substituenten A, B, X und Y jeweils unabhängig voneinander ausgewählt aus Wasserstoff, Hydroxyalkyl (worin die Alkylgruppe 2 bis 6 Kohlenstoffatome enthält), Methylenphosphon-, Methylen-, Ethylen- und Propylensulfon-, Hydroxymethyl-, Hydroxyethyl- und Hydroxypropyl-Sulfonsäurereste, Carbonsäurereste (mit 2 bis 4 Kohlenstoffatomen) und die Alkali- oder Erdalkalimetall-, Ammonium- und Aminsalze von einer der Phosphon-, Sulfon- oder Carbon-Säurederivate sind, und worin mindestens eines von A, B, X und Y von Wasserstoff verschieden ist.

2. Verbindung nach Anspruch 1, worin mindestens eines von A, B, X und Y ein Methylenphosphonsäurerest oder ein Salz davon ist.

3. Verbindung nach Anspruch 2, worin jedes von A, B, X und Y ein Methylenphosphonsäurerest oder ein Salz davon ist.

4. Verbindung nach Anspruch 2, worin eines von drei von A, B, X und Y ein Methylenphosphonsäurerest oder ein Salz davon ist und das verbleibende Wasserstoff ist.

5. Verbindung nach Anspruch 2, worin jedes von drei von A, B, X und Y ein Methylenphosphonsäurerest oder ein Salz davon und das verbleibende eine ein 2-Hydroxypropyl-2-sulfonsäurerest oder ein Salz davon ist.

6. Verbindung nach Anspruch 2, worin jedes von drei von A, B, X und Y ein Methylenphosphonsäurerest oder ein Salz davon und das verbleibende eine ein Carboxymethylrest oder ein Salz davon ist.

7. Verbindung nach Anspruch 2, worin jedes von drei von A, B, X und Y ein Methylenphosphonsäurerest oder ein Salz davon und das verbleibende eine ein 2-Hydroxypropylrest ist.

8. Verbindung nach Anspruch 1, worin jedes von A, B, X und Y ein Carboxymethylrest oder ein Salz davon ist.

9. Verbindung nach einem der Ansprüche 2 bis 8, worin alle Säuregruppen in Salzform vorliegen.

10. Verbindung nach einem der Ansprüche 2 bis 9, in Form von Alkalimetall- oder Erdalkalimetallsalzen.

11. Verbindung nach Anspruch 10, worin das Salz ein Natriumsalz ist.

12. Verfahren zur Verhinderung der Präzipitation von Metallionen aus ihren wäßrigen Lösungen durch Zugabe eines Phosphonsäurederivats zu den Lösungen in weniger als den stöchiometrischen Mengen, bezogen auf die anwesenden Matallionen, dadurch gekennzeichnet, daß das Derivat eine Verbindung nach einem der Ansprüche 2 bis 7 und 9 bis 11 ist.

13. Verfahren zur Chelatierung von Metallionen in einer wäßrigen Lösung dieser Ionen durch Zugabe

von mindestens einer stöchiometrischen Menge, auf Basis der anwesenden Metallionen, eines Aminderivats, dadurch gekennzeichnet, daß das Derivat eine Verbindung nach einem der Ansprüche 1 bis 11 ist.

14. Verfahren zur Verzögerung des Absetzens einer wäßrigen Zementaufschlämmung, umfassend Zugeben zu der Aufschlämmung eines organischen Phosphonats, dadurch gekennzeichnet, daß das Phosphonat eine Verbindung nach einem der Ansprüche 2 bis 7 und 9 bis 11 ist.

15. Verfahren nach Anspruch 14, worin die Temperatur der Zementaufschlämmung mindestens 80°C (180°F) ist.

16. Verfahren nach Anspruch 14, oder 15, worin die Zementaufschlämmung in ein Ölbohrloch eingespritzt wird.

**Revendications**

1. Composé de formule

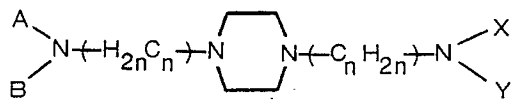

dans laquelle n est égal à 2 ou 3 et dans laquelle les substituants A, B, X et Y sont chacun choisis indépendamment parmi un hydrogène, les radicaux hydroxyalkyle (où le groupe alkyle contient 2—6 atomes de carbone), acide méthylène-phosphonique, méthylène-, éthylène- et propylène-sulfonique, hydroxyméthyl-, hydroxyéthyle- et hydroxypropylsulfonique, les radicaux acide carboxylique (possédant 2—4 atomes de carbone) et les sels de métaux alcalins ou alcalino-terreux, d'ammonium et d'amine de n'importe quel dérivé acide phosphonique, sulfonique ou carboxylique, et dans laquelle l'un au moins de A, B, X et Y est autre qu'un hydrogène.

2. Composé selon la revendication 1, dans lequel l'un au moins de A, B, X et Y est un radical acide méthylène-phosphonique ou un de ses sels.

3. Composé selon la revendication 2, dans lequel chacun de A, B, X et Y est un radical méthylène-phosphonique ou un de ses sels.

4. Composé selon la revendication 2, dans lequel trois parmi A, B, X et Y sont chacun un radical acide méthylène-phosphonique ou un de ses sels, et le restant est un hydrogène.

5. Composé selon la revendication 2, dans lequel trois parmi A, B, X et Y sont chacun un radical acide méthylène-phosphonique ou un de ses sels, et le restant est un radical acide 2-hydroxypropyl-3-sulfonique ou un de ses sels.

6. Composé selon la revendication 2, dans lequel trois parmi A, B, X et Y sont chacun un radical acide méthylène-phosphonique ou un de ses sels, et le restant est un radical carboxyméthyle ou un de ses sels.

7. Composé selon la revendication 2, dans lequel trois parmi A, B, X et Y sont chacun un radical acide méthylène-phosphonique ou un de ses sels, et le restant est un radical 2-hydroxypropyle.

8. Composé selon la revendication 1, dans lequel chacun de A, B, X et Y est un radical carboxyméthyle ou un de ses sels.

9. Composé selon l'une quelconque des revendications 2 à 8, dans lequel tous les groupes acides sont sous forme d'un sel.

10. Composé selon l'une quelconque des revendications 2 à 9, sous forme d'un sel de métal alcalin ou de métal alcalino-terreux.

11. Composé selon la revendication 10, dans lequel le sel est un sel de sodium.

12. Procédé pour inhiber la précipitation d'ions métalliques dans leurs solutions aqueuses, qui consiste à ajouter auxdites solutions, et quantités inférieurs à la quantité stoechiométrique, par rapport aux ions métalliques présents, un dérivé acide phosphonique, caractérisé en ce que ledit dérivé est un composé selon l'une quelconque des revendications 2 à 7 et 9 à 11.

13. Procédé pour chélater des ions métalliques dans une solution aqueuse de ceux-ci, qui consiste à y ajouter au moins une quantité stoechiométrique, par rapport aux ions métalliques présents, d'un dérivé amine, caractérisé en ce que ledit dérivé est un composé selon l'une quelconque des revendications 1 à 11.

14. Procédé pour retarder la prise d'une suspension aqueuse de ciment, qui comprend l'addition à ladite suspension d'un phosphonate organique, caractérisé en ce que ledit phosphonate est un composé selon l'une quelconque des revendications 2 à 7 et 9 à 11.

15. Procédé selon la revendication 14, dans lequel la température de la suspension de ciment est d'au moins 80°C (180°F).

16. Procédé selon la revendication 14 ou 15, dans lequel on injecte la suspension de ciment dans un puits de pétrole.